# EUROPEAN PATENT APPLICATION

(11) **EP 1 158 058 A1**
(43) Date of publication of application: **28.11.2001**
(21) Application number: 00401372.8
(22) Date of filing: 19.05.2000
(51) Int. Cl.: C12Q 1/68

(54) **Compositions and methods suitable for nucleic acid analyses**

(71) Applicant: CENTRE NATIONAL DE LA RECHERCHE SCIENTIFIQUE, 75016 Paris Cédex (FR)
(72) Inventor: Dumas, Sylvie, 75006 Paris (FR); Vujasinovic, Todor, 75007 Paris (FR); Mallet, Jacques, 75013 Paris (FR)
(74) Representative: Becker, Philippe

(57) **Abstract**

The present invention relates to compositions and methods for nucleic acid analyses. More particularly, this invention provides compositions and methods for differential gene expression analyses on nucleic acid arrays. This invention discloses more preferably differential gene expression analyses on nucleic acid arrays using nucleic acid samples having distinct radioactive labels Even more particularly, this invention relates to compositions and methods for nucleic acid analysis, comprising contacting at least two differently radiolabelled nucleic acid samples on a nucleic acid array, and detecting (or comparing or quantifying) hybrids formed between the nucleic acids of the samples and the nucleic acid array. The present invention can be used to detect or monitor gene expression or to compare gene expression (e.g., differential gene expression screening), for instance, and is suitable for use in research, diagnostic and many pharmacogenomics applications, for instance.

## Description

### FIELD OF INVENTION

The present invention relates to compositions and methods for nucleic acid analyses. More particularly, this invention provides compositions and methods for differential gene expression analyses on nucleic acid arrays using nucleic acid samples having distinct radioactive labels. Even more particularly, this invention relates to compositions and methods for nucleic acid analysis, comprising contacting at least two differently radiolabelled nucleic acid samples on a nucleic acid array, and detecting (or comparing or quantifying) hybrids formed between the nucleic acids of the samples and the nucleic acid array. The present invention can be used to detect or monitor gene expression or to compare gene expression (e.g., differential gene expression screening), for instance, and is suitable for use in research, diagnostic and many pharmacogenomics applications, for instance.

### BACKGROUND

Nucleic acid arrays have been described in the art as a means to detect, quantify, screen, monitor or compare nucleic acid samples. Nucleic acid arrays are essentially composed of nucleic acids (targets) attached to a support, preferably in discrete, organized fashion. Nucleic acid arrays may be high density arrays (microarrays) or low density arrays (macroarrays). The nucleic acid targets attached to the support may be synthetic oligonucleotides or biological nucleic acids (such as gene fragments, RNA molecules, PCR products, PNAs, etc). The nucleic acid arrays can be contacted with various nucleic acid populations (probes) to be analysed (RNAs, mRNAs, DNAs, cDNAs, gDNAs, pre-selected populations thereof, etc). Through hybridisation, specific nucleic acids can be detected or differences between nucleic acid samples may be evidenced and characterized.

The use of high-density probe arrays in large-scale gene expression screenings faces several technological challenges. One of them is obtaining reproducible analysis of tissues or cell populations that are available only in very low quantity, such as cells obtained by needle biopsy or specific rat brain structures. A second one is gaining the required signal detection sensitivity to reproducibly analyse the expression of DNAs or messenger RNA species that may be expressed down to only a few copies per cell, or even to one copy per cell only. A third one, technically linked with the previous one, is gaining the ability to detect low modulations of gene expression (down to 30%), because such modulations may be of major biological significance. These three challenges are both of major scientific importance, because in most biological fields the samples under study are often difficult to obtain in large quantities, and because many genes of major scientific and/or pathological interest are expressed only at low levels, as opposed to a large number of domestic genes. Furthermore, the ability to better design models and understand complex biological systems depends upon the technological capacity to detect and quantify, in the same sample and during the same experiment, several messenger RNA species whose expression levels may be distributed in a 10⁴ to 10⁵ range. In areas like diagnosis, the same challenges and problem arise as biological samples of interest may be in very low quantity (e.g., bacteria in water, virus in a sample, etc.). All these challenges directly address the question of signal-detection sensitivity.

To this date, all standard nucleic acid microarray protocols, in particular high-density microarray protocols, require the use of fluorescence-labelled probes, the hybridisation result analysis being performed by a laser device. With such a detection system, and using a procedure in which two nucleic acid (e.g., RNA) samples to be compared are labelled with a different fluorescence dye and then simultaneously hybridised on the same array, it is possible to perform large-scale differential gene expression screenings. However, the current use of fluorescence labelling shows relatively poor performances in terms of signal detection sensitivity, making such a labelling not fully suitable for the requirements of microarray-based large-scale gene expression screenings in numerous biological fields such as neuronal plasticity. The same constraints and problems are encountered for procedures in which one nucleic acid sample is used in combination with control nucleic acids, for instance for diagnostic purposes.

The present invention provides novel compositions and methods that overcome the drawbacks of prior art techniques. In particular, the present invention provides methods and compositions for analysing nucleic acids that ensure high sensitivity, reproducibility and suitable through put for large screenings.

### SUMMARY OF THE INVENTION

More particularly, the present invention discloses alternative nucleic acid analysis methods based on radioactive labelling of the probes. This invention relates, more particularly, to compositions and methods for nucleic acid analysis, comprising contacting at least two differently radiolabelled nucleic acid samples on a nucleic acid array, and detecting (or comparing or quantifying) hybrids formed between the nucleic acids of the samples and the nucleic acid array.

This invention is more particularly based on the new concept of using radioactive labelling of the probes in nucleic acid array-based differential gene expression screenings. The present invention shows that differently radiolabelled nucleic acid samples can be produced and hybridised (simultaneously) to a nucleic acid array, and that differences between the samples can be evidenced by assessing radioactivity on the array. This invention demonstrates that samples can be produced in a way that allows the discrimination of fine gene variations based on specific detection of radioelements.

The invention provides methods and compositions for simultaneous analysis and/or comparison of nucleic acid samples comprising the detection and/or discrimination and/or quantification of target nucleic acids on a nucleic acid array, using radioactive labels.

The invention provides methods and compositions for simultaneous visualization and/or quantification and/or detection and/or discrimination of several nucleic acids in a at least two nucleic acid samples, comprising the simultaneous hybridisation of the nucleic acid samples to a nucleic acid array, said two nucleic acid samples being differently radiolabelled, and the detection and/or quantification and/or comparison of the radiolabel present on the array, corresponding to each nucleic acid sample.

The invention more specifically uses several nucleic acid samples that exhibit different (distinguishable) radiolabels for simultaneous hybridisation on a nucleic acid array, more preferable a high density nucleic acid array.

The present invention discloses, for the first time, methods that allow co-detection and quantitative analysis of gene expression using radioactive probes. This invention more particularly discloses that it is possible to differentiate gene expression and detect fine gene regulations using radioactive probes that are exposed simultaneously on the same nucleic acid array. This invention further shows that radioactive labeling provides increased sensitivity as compared to prior art methods, high reproducibility, and allows the detection (and quantification) of nucleic acid present at very low copy numbers in a sample, with no need for any nucleic acid amplification step.

The instant invention describes more specifically the simultaneous hybridisation and visualization of two radioactive probes on the same nucleic acid array, each probe being labelled with different radio-elements (³³P/³⁵S/³H/³²P/¹²⁵I, etc.). Taking in consideration the specific activity difference between various radiolabelled nucleotides, the invention also discloses preferred methods and conditions allowing the use of these different radioactive nucleotides to differently label different nucleic acid samples that would be hybridised on the same array (or biochip) and efficiently discriminate the probes on the same array.

A particular aspect of this invention resides in a method of nucleic acid analysis, comprising contacting at least two differently radiolabelled nucleic acid samples on a nucleic acid array, and analysing nucleic acids in the samples by detecting hybrids formed between the nucleic acids of the samples and the nucleic acid array.

Another aspect of this invention is a method of nucleic acid analysis, comprising:
a) providing a first nucleic acid sample labelled with a first radiolabel,
b) providing a second nucleic acid sample labelled with a second radiolabel, the second radiolabel being different from the first radiolabel,
c) contacting the first and second nucleic acid samples on a nucleic acid array, and
d) analysing nucleic acids in the samples by detecting hybrids formed between the nucleic acids of the samples and the nucleic acid array.

In further preferred embodiments, the first and/or second nucleic acid samples are DNA samples, in particular cDNA samples, even more preferably cDNA samples produced by reverse transcription of RNA populations, more particularly mRNA populations. In particular variants, the RNAs or mRNAs derive from different biological samples or from a same type of biological sample in a different physio-pathological condition. In other preferred embodiments, at least one of said nucleic acid samples is a gDNA sample, the other sample being composed of other nucleic acids, including control nucleic acids.

According to other preferred embodiments, the nucleic acid samples are labelled with radiolabels having a different emission-energy spectra, as an example, the first nucleic acid sample is labelled with tritium and the second nucleic acid sample is labelled with a radioisotope selected from ³⁵S, ³³P, ³²P and ¹²⁵I.

Preferably, the DNA samples are radiolabelled by incorporation of radiolabelled nucleotides in their sequence during reverse transcription or by other techniques such as linear PCR amplification, for instance.

Other preferred variants provide that the two samples are contacted simultaneously with the nucleic acid array, and/or the two samples have essentially the same specific activity and/or essentially the same amount of the two samples is used.

As will be further explained below, the nucleic acid array generally comprises, immobilized on a support, such as glass, nylon, plastic, gold, silicium or combinations thereof, single- or double-stranded nucleic acids selected from oligonucleotides, DNA, RNA, gene fragments, PCR products, Peptide Nucleic Acids ("PNAs") or combinations thereof. More particular examples of target nucleic acids include genomic DNA, DNA from cellular organelles and, more generally, DNA or any nucleic acid clone producible through molecular biology techniques or other technologies or obtainable from nucleic acid libraries. Specific examples of such clones include artificial chromosomes from yeast (YAC), baculoviruses (BAC), etc.

Other aspects of this invention reside in:
- a method of nucleic acid analysis, comprising:
   a) preparing a first cDNA sample labelled with a first radiolabel by reverse transcription of a first RNA population in the presence of a radiolabelled nucleotide labelled with the first radiolabel,
   b) preparing a second cDNA sample labelled with a second radiolabel by reverse transcription of a second RNA population in the presence of a radiolabelled nucleotide labelled with the second radiolabel,
   c) exposing the first and second cDNA samples to a nucleic acid array, and
   d) analysing nucleic acids in the samples by detecting hybrids formed between the nucleic acids of the samples and the nucleic acid array.
- a method for comparing at least two nucleic acid samples, comprising:
   a) labelling a first nucleic acid sample with a first radiolabel,
   b) labelling a second nucleic acid sample with a second radiolabel, said first and second radiolabels having a different emission energy spectra,
   c) simultaneously exposing at least a portion of said differently radiolabelled nucleic acid samples to a nucleic acid array under conditions allowing hybridisation to occur, and
   d) comparing the nucleic acid samples by analysing hybridisation pattern thereof.
- the use of at least two differently radiolabelled nucleic acid samples for in vitro gene expression analysis on a nucleic acid array.
- a method of preparing a radiolabelled nucleic acid sample, comprising:
   (a) obtaining RNAs from a biological sample, preferably mRNAs, more preferably using polyT-coated support, and
   (b) reverse transcribing the RNAs in the presence of a tritiated nucleotide, in order to produce tritiated cDNAs having incorporated in their sequence tritiated nucleotides.

The invention also encompasses kits for nucleic acid detection comprising radioactive nucleotides, enzymes and/or protocols for radioactive labelling of nucleic acid samples as well as, more generally, any kit for implementing a method as defined above, comprising the reagents, supports and/or protocols for labelling, hybridisation and/or readout.

As will be further demonstrated, this invention now shows that radioactive labelling is highly suitable for a number of gene expression screenings and/or gene detection (e.g., medical diagnostic of the presence of a bacteria, virus, genomic alteration, genotyping, karyotyping, etc.) on microarrays. It permits the performance of simultaneous hybridisation of two probes, with the highest signal detection sensitivity available to this date. It allows expression profiling experiments using sub-microgram amounts of un-amplified polyA-RNAs from small biological samples, with the possibility to detect even very low-expressed mRNAs. In addition, ³H-labelling is fully detected on (glass-support) microarrays, allowing competitive screening procedures to be performed by comparing ³H and either ³³P or ³⁵S or ³²P, for instance. The 5-µm pixel size of the MicroImager is satisfactory for microarray analysis. About 10,000 spots can be analysed on a same array with radioactive labelling. Considering the high absolute signal detection sensitivity and the low background of this technique, it should theoretically make possible the reproducible detection of less than 2-fold gene expression modulations of low-expressed genes.

### DETAILED DESCRIPTION OF THE INVENTION

As indicated, the present invention resides in methods of detecting or analysing gene expression or regulation using radiolabeled nucleic acid samples that are exposed to or contacted with a nucleic acid array. The present invention will now be disclosed in further details, the details being merely illustrative and not limiting the scope of the invention.

### 1. The Nucleic acid array

As indicated above, the nucleic acid array is generally composed of nucleic acids (targets) immobilized on a support, for instance in discrete, organized fashion. The array may also be designated biochip or nucleic acid chip, for instance. The array may be a high density array, comprising above about 20 000 nucleic acid molecules per cm square. It may also be a low density or moderate density array, with a nucleic acid density below the above numbers.

The nucleic acids on the array may be of various nature, including double- or single-strand DNA, RNA, cDNA, gDNA, gene fragments, PCR products, ESTs, oligonucleotides, PNAs, etc., including any combinations thereof, from any biological, synthetic or semi-synthetic origin. In particular, the nucleic acids on the array may be isolated directly from biological tissues or from libraries, they may be modified, or artificially synthesized, or produced by combinations of such methods. The nucleic acids on one array may be selected for any specific property of interest, such as (average) length, (type of) activity, biological origin, etc. Alternatively, they may be random oligonucleotides.

The nucleic acids may be immobilized to the support using various techniques and strategies. In a particular embodiment, the nucleic acids are synthesized directly on the support ("in situ synthesis"), by photolitography or other techniques as described for instance in Nature Genetics Suppl. 21, 1999 . This approach and these techniques are suitable for nucleic acid arrays comprising oligonucleotides of average length below 25 bases with predetermined or random sequence.

In another embodiment, the nucleic acids to be immobilized on the support are first produced (or prepared) and then attached to the support. Immobilization may be accomplished using various techniques disclosed in the art, allowing covalent attachment of nucleic acids to supports, either directly or through intermediate molecules (linkers), such as various types of polymers. In a preferred embodiment, the nucleic acid array comprises nucleic acids attached to a support via a linker molecule, more preferably a dendromeric linker molecule, as described in french patent application n° FR99 15967.

Typically, the nucleic acid array (or chip) comprises nucleic acids selected from oligonucleotides, gene fragments, PCR products, mRNAs, cDNA molecules or PNAs, attached to a support in organized fashion. More preferably, the array is a high density array comprising at least 20 000 nucleic acid molecules per cm square.

The support may be any suitable support for genetic analysis, including plastic, nylon, glass, gold, silicium, etc. The support is preferably solid (or semi-solid), such as a membrane or a slide, and has a surface allowing attachment of nucleic acids in conditions allowing hybridisation thereof with selected biological samples. Preferably, the support is a glass-derived support, i.e. comprises glass or any derivatized or functionalized component thereof. A more preferred support is a glass-containing slide, which allow fine and efficient analysis and discrimination of radioactive labels, as will be demonstrated below. A typical example of glass slide includes the SuperFrost^{R} Plus (Menzel-Glaser, Germany). Furthermore, the support may be pre-treated to ensure adhesion or immobilization of the nucleic acids and/or facilitate hybridisation step. Typically, the support is coated with poly-lysine, or silylated or silanated. Glass slides may be obtained from commercial sources such as Sigma, BDH, Menzel-Glaser, etc.).

Preferred examples of nucleic acid arrays or chips have been described in french patent application n° FR99 15967, incorporated therein by reference.

The support or the nucleic acid array (or biochip) may be used directly, or stored for later use.

### 2. The nucleic acid samples to be analysed

The present invention can be used to analyse virtually any type of nucleic acid preparation, i.e., of any origin, nature, diversity, etc.

Particularly, this invention discloses methods and compositions that can be used to compare at least two nucleic acid samples, in order to assess differences in gene expression or gene regulation. The nucleic acid sample may comprise DNA, gDNA, cDNA, RNA, fragments and/or combinations thereof, etc. The invention is also suitable to detect the presence or expression of (a) gene(s) or nucleic acid sequence(s) in any sample, including soil, water, tissue, food, drinks, etc. In this embodiment, the second nucleic acid sample may comprise one or several control genes (or nucleic acids).

In one embodiment, the invention uses at least two nucleic acid samples of essentially the same nature (i.e., DNA and DNA, RNA and RNA, etc), in particular, at least two DNA or cDNA nucleic acid samples.

In another embodiment, the at least two nucleic acid samples have a different nature (e.g., cDNA and gDNA, oligonucleotide and gDNA, for instance, etc.).

In a preferred embodiment, the nucleic acid samples are RNAs (such as total RNAs or mRNAs) or DNAs (in particular cDNAs) prepared from a biological sample, such as a cell, tissue, organ, biopsy, culture, etc. Even more preferably, the nucleic acid samples are cDNA samples prepared by reverse transcription of RNA populations isolated from biological samples as described in further details below.

### 2.1. The biological sample

The biological sample may be any mammalian biological material such as tissue sample, organ sample, biopsy, skin sample, biological fluid, bone marrow, nervous tissue (e.g., brain tissue), etc. The biological material may also comprise plant tissue or cells, prokaryotic cells, lower eukaryotic cells, established cell cultures, viruses, any other unicellular organism, etc. The biological sample may also include soil, water, tissue, food, drinks, air, gas, etc. Because of the high sensibility and high reproducibility of the present method, very low quantities of biological material may be used, and the invention can be applied to essentially all types of biological material. The invention is particularly suited for detecting rare mRNA species as well as fine gene expression regulation within complex tissues, such as nervous tissue. Preferably, the sample is a mammalian tissue sample, in particular a human tissue sample, such as nervous cells, blood cells, tumor cells, embryonic cells, etc.

The present invention is more particularly suited for comparing gene expression or regulation between a first biological sample and a second biological sample. The first and second biological samples may be essentially of the same nature (e.g. same type of cells or tissue, etc.) but in a different phyio-pathological condition, thereby allowing to analyse or compare (or detect) nucleic acids or nucleic acid regulations characteristic of a given condition (e.g. pathology vs healthy, proliferating vs quiescent, etc.). Alternatively, the biological samples may also be of different nature or origin, extending the utility of the present invention to the differential analysis of any nucleic acid samples.

The invention is also suitable for detecting any nucleic acid in a sample, by hybridisation of the labelled sample with the nucleic acid array, in the presence of one or several control nucleic acids having a distinct radiolabel.

### 2.2. Preparation of nucleic acid samples from a biological sample

As indicated above, while any nucleic acid sample is convenient for use in the present invention, the nucleic acid samples are preferably RNAs, DNAs or cDNAs prepared from a biological sample. Various conventional techniques may be used to isolate and prepare DNAs, RNAs or cDNAs from a biological sample.

### 2.2.1. RNA extraction

RNAs may be prepared by various known preparative methods using solvants and/or chromatographic and/or affinity methods. In a preferred embodiment, RNAs are recovered (or isolated) from the biological sample by treatment of the biological sample to release the nucleic acids from cells (lysis, detergent, sonication, enzymatic digestion, etc.), followed by separation of total or messenger RNAs therefrom. RNAs can be isolated according to known techniques such as solvent extraction. Messenger RNAs can be isolated from the biological sample or from total RNAs based on the presence of a polyA tail at the 3' end of each messenger RNA.

In a particular embodiment, the mRNAs are obtained by contacting the above treated biological sample with polyT-coated support. The mRNAs attach to the support through hybridisation and can be released therefrom under appropriate saline conditions. The polyT more preferably comprises, on average, between about 5 and about 50 bases, more preferably between about 5 and about 40. The polyT-coated support may comprise beads, column, plates, etc., more preferably poly-T coated beads or columns. PolyT-coated beads can be obtained from commercial sources, such as from Dynal (oligo(dT)₂₅, 610.02). Typically, the beads are magnetic beads which can be recovered by applying a magnetic field. OligodT columns include cellulose-oligodT columns, available for instance from Pharmacia (oligo(dT)cellulose type 7 or type 77F), Boehringer, etc. It should be understood that any other isolation method or device may be used for preparing RNAs without departing from the present invention.

In an other embodiment, the mRNAs are not isolated and cDNA production is performed using total RNAs.

Finally, where the nucleic acid samples are DNAs, they may be prepared by any conventional techniques, including the use of chromatographic columns such as resin columns (Promega, etc.).

### 2.2.2. cDNA production

cDNAs can be prepared from RNAs (or mRNAs) using conventional techniques. They may also be obtained directly from libraries or other preparations available. More particularly, the cDNAs are prepared by reverse transcription of RNAs in the presence of a primer (generally a poly(dT) molecule), nucleotides and a reverse transcriptase. The respective amounts or concentrations of RNAs, primer, nucleotides and reverse transcriptase may be adjusted by the skilled person, as well as the temperature and duration time of the reaction. Typically, about 10ng to about 100µg RNAs are incubated with an excess of poly(dT) primer, to ensure annealing of poly(dT) with essentially all polyA-tailed RNA species (or molecules) present in the sample. In a specific embodiment, about 100ng to about 10µg RNAs are incubated with 0.5µg to 50µg poly(dT) primer. Furthermore, to facilitate or increase the efficiency of annealing, the mixture may be subjected to heating (to about 60-80°C for instance) and progressively cooled (to about 40-50°C for instance).

cDNA synthesis can then be performed in the presence of essentially similar concentrations of each nucleotide (e.g., between about 0.1 to about 5 mM, more preferably between about 0.1 to about 2 mM) and sufficient amounts of reverse transcriptase, typically between about 0.01 to about 10 Units/µl. Particular reverse transcriptase that can be used in this reaction include AMV RT (Prolabo), M-MLV reverse transcriptase (Promega), etc.

As will be further explained below, in order to produce radiolabelled cDNA samples, the reverse transcription reaction may be performed in the presence of at least one (preferably only one) radiolabelled nucleotide, that is incorporated into the cDNA molecules. Each cDNA sample may thus be prepared by reverse transcription in the presence of (a) particular radiolabelled nucleotide(s), thereby providing each sample with a particular radiolabel. For instance, one sample may be prepared by reverse transcription in the presence of a tritiated nucleotide selected from A, C, T and G, the remaining three nucleotides being non-radiolabelled, and the other sample may be prepared by reverse transcription in the presence of a phosphorated or iodinated or thio-labelled nucleotide selected from A, C, T and G, the remaining three nucleotides being non-radiolabelled.

In a particular embodiment, the reverse transcription reaction is performed at a temperature comprised between about 35 to about 50°C, more preferably between about 38 to about 45°C. The reaction can last for about 10 minutes to about 5 hours, for instance. It should be understood that these parameters can be adjusted easily by the skilled person.

It is preferred that no amplification step occurs or is performed on the RNA sample. Indeed, the present invention now provides methods and compositions allowing detection (and quantification) of virtually any nucleic acid species in a sample, including those present at very low concentration. The invention thus allows the direct analysis of nucleic acid samples from biological tissues with no need for nucleic acid amplifications which are known to potentially alter the respective amounts and diversity of nucleic acid molecules in a sample.

Upon reverse transcription, RNAs may be removed from the reaction product by conventional techniques, as well as unincorporated nucleotides (for instance on a P10 chromatography column). The resulting preparation, or aliquots thereof, can be used in genetic analyses methods according to the present invention, or stored for subsequent uses.

### 2.3. Nucleic acid labelling

As indicated above, this invention resides in the use of radioactive nucleic acid populations, more specifically nucleic acid samples having distinct radioactive labels, in order to detect and monitor fine gene expression and regulation.

More preferably, the invention uses at least two nucleic acid samples which are differently radiolabelled, e.g., labelled with particular radioelements which can be distinguished from each other.

### 2.3.1. Radiolabel

Many radio-elements or isotopes can be used for the labelling of the samples. Specific examples of isotopes include ³H, ³⁵S, ³³P, ³²P, ¹⁴C, ¹²⁵I, and the like.

Preferably, the invention uses at least two samples as defined above, the samples being labelled with radioelements having a different emission energy, more preferably a distinguishable emission energy spectra. More preferably, the mean emission energy of the radioelements used should differ of at least 10 Kev, more preferably at least 20 Kev, even more preferably at least 30 Kev. Table 1 below discloses the emission energy, resolution and period for the preferred radioelements to be used in this invention.

**TABLE 1**

| Radioisotopes | emission | mean energy (KeV) | max. energy (KeV) | resolution (µm) | period |
|---|---|---|---|---|---|
| ³H | β⁻ | 5.7 | 18.6 | 0.5-5 | 12.3 years |
| ¹⁴C | β⁻ | 49.4 | 156.5 | 10-20 | 5730 years |
| ³⁵S | β⁻ | 48.8 | 167.5 | 10-15 | 87.4 days |
| ³³P | β⁻ | 76.4 | 248.5 | 15-20 | 25.6 days |
| ³²P | β⁻ | 695.5 | 1710.4 | 20-30 | 14.3 days |
| ¹²⁵I | e⁻ auger | 3.7 (79.3) | | 1-10 | 59.9 days |
| | | 22.7 (19.9%) | | | |
| | | 30.6 (10.7%) | | | |
| | | 34.5 (3.3%) | | | |
| | γ | 35.5 (6.7%) | | | |
| | X | 27.2 (39.6%) | | | |
| | | 27.4 (73.8%) | | | |
| | | 30.9 (21.3%) | | | |
| | | 31.7 (4.3%) | | | |

Table 1 shows that ³H emission energy spectrum is clearly distinguishable from that of ³⁵S, ³³P, ³²P and ¹²⁵I, for instance. In a preferred embodiment, one nucleic acid sample is thus labelled with tritium and another nucleic acid sample is labelled with a radioisotope selected from ³⁵S, ³³P, ³²P and ¹²⁵I. The examples disclosed below provide evidence that such sets of differently labelled nucleic acid samples can be used efficiently to simultaneously detect and discriminate target nucleic acids on a same array, with a very high sensitivity.

Radioactive nucleotides to be used in this invention include natural and non-natural radiolabelled nucleotides, more preferably radiolabelled nucleotides selected from ATP, dATP, CTP, dCTP, GTP, dGTP, UTP, dUTP, TTP, dTTP. Such nucleotides are commercially available, or may be produced by conventional chemical methods. More preferred radiolabelled nucleotides to be used in the instant invention are listed in Table 2 below:

**TABLE 2**

| Isotope | Nucleotide | ref. | specif. Activity Ci/mmole |
|---|---|---|---|
| ³H | dATP | TRK 633 | 50-100 |
| | | TRK 347 | 1-10 |
| | dCTP | TRK 625 | 50-85 |
| | | TRK 352 | 15-30 |
| | dGTP | TRK 627 | 25-50 |
| | | TRK 350 | 5-20 |
| | dUTP | TRK 351 | 5-30 |
| ³⁵S | d ATPαS | SJ 1304, 304,264, 1334, 1300 | 400-1000 |
| | d CTPαS | SJ 1305, 305, 1302 | 400-1000 |
| | UTPαS | SJ 1303, 603, 263 | 400-1000 |
| ³³P | (γ) ATP | BF1000 | ≥2500 |
| | (α) dATP | BF1001 | ≥2500 |
| | (α) dCTP | BF1003 | ≥ 2500 |
| | (α) CTP | BF1012 | ≥ 2500 |
| | (α) UTP | BF1002 | ≥ 2500 |
| ³²P | (α) dATP | PB10474, 10204, 10384, 10164 | 400-6000 |
| | (α) ATP | PB10200, 10160 | 400-3000 |
| | (γ) ATP | PB218, 168, 10218, 10168 | 3000-5000 |
| | (α) ddATP | PB10235, 10233 | 3000 - > 5000 |
| | (α) dCTP | PB10475, 10205, 10385, 10165 | 400-6000 |
| | (α) CTP | PB10202, 20382, 10162, 40382 | 400-3000 |
| | (α) dGTP | PB10206, 10386, 10166 | 400-3000 |
| | (α) GTP | PB10201, 10161 | 400-3000 |
| | (γ) GTP | PB10244 | > 5000 |
| | (α) dTTP | PB10207, 10387, 10167 | 400-3000 |
| | (α) UTP | PB10163, 10203, 20383 | 400-3000 |
| ¹²⁵I | dCTP | NEX 074 | 2200 |

Even more preferably, radiolabelled nucleotides with high specific activity are being used, in order to produce samples with high specific activity value, as will be further disclosed below.

The nucleic acid samples may be radio-labelled according to different techniques.

### 2.3.2. Labelling during synthesis

In a preferred embodiment, the nucleic acid samples are labelled during their synthesis. In this embodiment, radiolabelled nucleotides are incorporated into the samples during the synthesis. This embodiment is particularly suited for RNA samples which are produced in in vitro transcription systems, or for cDNA samples prepared by reverse transcription from RNA preparations.

The specific activity of the sample can be adjusted by selecting the radionucleotide having a particular specific activity (see Table 2 above) as well as by controlling the concentration of radiolabelled nucleotide in the synthesis medium.

In a preferred embodiment, the nucleic acid sample is a cDNA sample prepared by reverse transcription of a RNA preparation in the presence of a radionucleotide. In a more preferred embodiment, the radionucleotide is labelled with a radioisotope selected from ³H, ³⁵S, ³³P, ³²P and ¹²⁵I. Even more preferably, one nucleic acid sample is a cDNA sample prepared by reverse transcription of a RNA preparation in the presence of a tritiated nucleotide and another nucleic acid sample is a cDNA sample prepared by reverse transcription of a RNA preparation in the presence of a radionucleotide labelled with a radioisotope selected from ³⁵S, ³³P, ³²P and ¹²⁵I.

In a preferred embodiment, each nucleic acid sample to be used in the same assay should be labelled using the same technique (i.e., post-synthesis or during synthesis, 3' tail vs 5' phosphate, etc).

### 2.3.3. Post-synthesis (or extraction) labelling

In an other embodiment, the samples may be labelled post-synthesis. In this embodiment, the samples are first produced and then labelled, using a selected radio-isotope.

Post-synthesis labelling may be performed according to various strategies. In a particular variant of this invention, the samples are labelled by addition of a terminal radioactive tracer thereto. In a more preferred embodiment, the terminal radioactive tracer comprises one or several radioactive nucleotides having the same radio-isotope, i.e., a radioactive tail. The tail may be a homopolymer, i.e., composed of the same repeated nucleotide, or a heteropolymer, i.e., composed of several different nucleotides. Where a heteropolymer tail is used, the sequence should preferably be determined so as not to interfere with the hybridisation of the nucleic acid sample and not to form secondary structures (loops, etc.).

In a preferred embodiment, the terminal radioactive tracer is a homopolymer tail, more preferably a 3'(homopolymer)-tail.

Furthermore, in the tail, all or only a part of the nucleotides may be radio-labelled. Indeed, by adapting the concentration or proportion of radioactive nucleotides in the tail, it is possible to control or adjust the specific activity of the nucleic acid sample. Obviously, the radioactive nucleotides present in the tail should preferably all bear the same radio-isotope so that each nucleic acid sample is characterized by a particular radioisotope.

The specific activity of the nucleic acid samples may be further adapted by controlling or adjusting the length of the tail. In this regard, in a particular embodiment of this invention, the tail comprises preferably 5 to 100 nucleotides, more preferably between 5 and 50 nucleotides, even more preferably between 5 and 30 nucleotides, and even more preferably at least 25% of the nucleotides in the tail are radiolabelled.

The tail may be produced either separately and then linked to the nucleic acids in each sample, or by direct sequential addition of the nucleotides to the nucleic acids in the samples.

In this regard, in a particular embodiment, the nucleic acid sample is labelled by contacting the nucleic acid sample with radioactive nucleotides in the presence of an enzyme that catalyses the 3' binding of nucleotides. A typical enzyme to be used is a terminal transferase. As indicated above, the concentration of the nucleotides and the proportion of radioactive and non radioactive nucleotides may be adapted to adjust the specific activity of the nucleic acid sample.

In a more particular variant, the nucleic acid sample comprises a 3'-tail produced by sequential addition to the probe of 5-100 nucleotides, all or part of which bearing a selected radiolabel. More preferably, the 3' tail is a 5-100 bases long homopolymer, preferably a polyA, polyC, polyG, polyT or polyU tail, in which all or part of the nucleotides bear a selected radioisotope.

Post synthesis labelling may also be performed by addition of radiolabelled phosphates (e.g., (γATP, γGTP)³²P, γATP³³P, ³⁵S-thio-phosphates) to the 5' end of the nucleic acids in each sample, using suitable enzymes such as T4 kinase. Such method may be used alone or in combination with others, since it may not allow very high specific activity to be achieved.

### 2.3.4. Non-radioactive probes or labelling

While the invention uses at least two differently radiolabelled nucleic acid samples, it should be understood that the invention may be performed by combining said radiolabelled samples with any other samples, including non-radioactive samples such as fluorescent samples, so that additional genes or RNAs can be monitored simultaneously.

### 3. Hybridisation

The present invention now provides, for the first time, evidence that differently labelled nucleic acid samples (NAS) can be contacted or exposed simultaneously on a nucleic acid array (NAA) and that the signals emitted can be discriminated, thereby allowing to monitor and quantify gene expression or gene regulations. The invention also demonstrates that improved discrimination can be made by adapting the specific activity of the NAS and controlling the hybridisation conditions, as will be discussed below.

In the present invention, the NAA is contacted with at least two NAS as defined above. The contacting allows formation of hybrids between the nucleic acids of the samples and the array. Accordingly, the contacting shall be made under conditions sufficient to allow nucleic acid hybridisation to occur. Conditions for forming hybridisation have been disclosed for instant in Maniatis et al (Molecular Cloning, a Laboratory Manual, 1989) or in Nucleic Acid Hybridization, A practical approach IRL Press, Wash. DC (1985).

In this regard, in order to ensure high sensitivity of the method, the contacting step is preferably performed under conditions allowing the nucleic acids of each sample to hybridise with their complementary (target) nucleic acid on the array. As hybridisation may also potentially occur with non-target (i.e., aspecific) nucleic acids, non-specific hybridisation can be eliminated or reduced by suitable washing conditions. The hybridisation condition can be adjusted by the skilled artisan. Essentially, hybridisation can be controlled by the hybridisation medium and temperature. In this respect, hybridisation is preferably performed at temperatures between about 30 and about 70 °C (more preferably between about 50 and about 70°C). Furthermore, the hybridisation medium generally comprises standard saline citrate solution (SSC) at moderate saline strength. Specific hybridisation conditions are disclosed in the examples and can be adapted by the skilled person. Typically, the hybridisation medium comprises SSC solution (1-5X) and, optionally, SDS (0.05-5X). Furthermore, the hybridisation medium may comprise additional agents that reduce non-specific signal or probes rearrangements, such as dithiothreitol (DTT) and/or formamide. In addition, in a particular aspect of this invention, hybridisation is performed in the presence of competitor nucleic acid, to reduce background signal. In particular, where the NAS contain a labelled nucleotide tail, the contacting step can be performed in the presence of un-labelled oligonucleotides complementary to the tail. The competitor nucleic acid may be used simulatenously with the NAS, or contacted with the array prior to the NAS.

Furthermore, prior to exposing the array to the NAS, the NAS may be heated and (progressively) cooled in order to eliminate or reduce secondary structures or intermolecular hybridisations.

In a typical experiment, the NAA is contacted with (or exposed to) a hybridisation medium in the presence of at least two radioactive NAS, for a period of time sufficient to ensure formation of hybrids, for instance between 1 hour to 24 hours, preferably between about 10-20 hours. The array may be covered with a film during hybridisation.

In order to allow efficient discrimination and visualization of radiolabelled nucleic acids on the array(s), it is preferred to use particular amounts of NAS, with a particular specific activity, for the hybridisation step. In this regard, the invention now demonstrates that efficient discrimination (and quantification) of the different labels is best achieved where both NAS have a specific activity comprised between about 5.10⁷ and 5.10¹⁰ cpm/µg, more preferably between about 10⁸ and 10¹⁰ cpm/µg, even more preferably between about 5.10⁸ and 5.10⁹ cpm/µg. A more preferred way of performing the invention comprises the use of two NAS having essentially the same specific activity, i.e., not differing by more than about 3 times from each other(s), more preferably not by more than about two times. The specific activity of the probes can be adjusted by the choice of the nucleotide (see table 2 above) and the conditions of the labelling method, as discussed above. In this respect, where the selected radionucleotides have a distinct specific activity, the labelling conditions should be adjusted to ensure that the labelled probes have essentially a similar specific activity.

In addition, in performing the hybridisation, it is also recommended to use similar amounts of each NAS, so that more reliable and comparable results are obtained. In this regard, typical experiments are performed using between about 1 and about 50 ng/µl of nucleic acids of each sample, more preferably between about 2 and about 20ng/µl. While these are preferred conditions allowing discrimination of nucleic acids present at very broad spectrum of levels (i.e., from rare to very abundant) and from virtually any type of biological material, it should be understood that the molarity (or amount) of nucleic acids of each sample can be adjusted by the skilled artisan to the specific conditions or biological samples.

In order to perform simultaneous analysis of differently radiolabelled NAS, each labelled NAS is contacted simultaneously with the array. The term "simultaneously" indicates that the hybridisation should be performed with the two NAS essentially at the same time, so that only one hybridisation/washing round in performed. However, "simultaneous" does not require that the NAS be contacted with the array at exactly the same time.

In a particular embodiment, the NAS are mixed with the hybridisation medium, and the array(s) is (are) then exposed to the resulting solution.

In another embodiment, the array(s) is(are) first exposed to the hybridisation medium, and the NAS are then added, either simultaneously or sequentially.

It goes without saying that the invention can be performed using either one single nucleic acid array or several nucleic acid arrays, sequentially or, preferably, in parallel.

Typically, between 20 to 200 µl of hybridisation medium is added to each array. The exposure time may vary, for instance, from 1 or several hours to one or several days. Preferably, the hybridisation lasts for less than about 24 hours, typically between 1 and 20 hours.

The arrays are then rinsed to eliminate unbound nucleic acids as well as non-specific hybridisation. In this regard, any conventional washing solution may be used, such as saline solutions. Preferably, the arrays are washed using saline citrate solution (SSC) comprising SDS, in order to eliminate non-specific hybrids formed. Preferred washing conditions use SSC supplemented with SDS (e.g., 0.1%) at room temperature. Several washings may be performed to increase the selectivity of the method.

The samples are then preferably apposed to scintillating paper for subsequent measure of the radioactivity (readout).

### 4. Readout

In order to assess hybrid formation on the arrays and to detect and discriminate the presence (or amount) of radiolabelled nucleic acids on said arrays, the method preferably comprises (i) washing the unbound nucleic acids (as described above) and (ii) detecting radioactivity (i.e., the first and second radiolabel) on the array(s).

Radioactivity detection and discrimination may be achieved by different techniques using quantitative imaging devices such as Beta Imager (50-250 µm depending on the radioisotope used) and the Micro Imager that provides direct detection by the solid scintillator sheet principle and allows resolution to fit with the size of the nucleic acid array (15 µm).

Preferably, acquisition of radioactive images is performed with a Micro Imager (Biospace Mesures, Paris, France), a real time, high-resolution digital autoradiography system. The instrument allows precise quantitative imaging of tissue section with a spatial resolution of 15 µm and a pixel size of 5 µm. Imaging is performed by optical contact between the radiolabeled sample, a thin foil of scintillating paper, and an intensified CCD camera. Beta particles are identified through light spot emission in the scintillating foil, allowing thus filtering of the background noise as well as filtering of emissions due to isotopes of different energies (FR2,772,484). The instrument is particularly well suited to the imaging and quantification of dual labelled samples and in particular to the simultaneous measurement of differential gene expression.

Imaging is performed on a 24 mm x 32-mm area. An automated sample feeder allows successive imaging of up to four slides. Detection threshold is kept to the very low level of 0,4 counts per minute per square millimetre for tritium labelling, and ten times lower for higher energy isotopes, a figure obtained thanks to the intrinsic noise suppression of the instrument. Because of the direct particle counting principle of the instrument, quantification is obtained with a precision better than 5%, without underexposure or saturation effects over four decades. Very fine variations of gene expression levels can therefore be measured with high accuracy.

In a preferred embodiment, radioactivity detection is thus performed by optical contact between the labelled sample, a thin foil of scintillating paper and an intensified CCD camera.

The invention can be used to monitor gene expression in any biological sample, for research, diagnostic or any other experimental or industrial applications (pharmacogenomics, etc). Gene expression may be used to identify a dysfunction, compare gene regulation, identify therapeutic genes, assess responsiveness of a subject, assess the presence of pathogenic agents (e.g., virus, bacteria, etc.) in a sample, etc. A particular advantage of this method is that it enables the use of tritium (³H) for radioactive detection of the hybridisation results. Tritium has never been used before in array experiments or assays, because its low-energy emission was considered as preventing any such application. The present invention now demonstrates that it is possible to use such a radioelement, in particular with solid supports where the probes and targets are only adsorbed on the surface of the support. In this respect, this invention also relates, generally, to the use of tritium for detecting nucleic acid hybridization on a nucleic acid array as well as to methods of nucleic acid analysis comprising a hybridisation of a nucleic acid sample on a nucleic acid array, wherein the nucleic acid sample is radiolabelled with tritium.

The results presented show that radioactive labelling has several advantages over fluorescent labelling :

First, we were able to use as little as 100 ng of polyA RNA for cDNA synthesis and still detect a mRNA expressed at less than 1 copy/500.000 of total mRNA, without any probe amplification. This corresponds to 5 mg of starting neural tissue. M. Mahadevappa and J.A. Warrington recently published a successful protocol of transcription-amplification of the probe, adapting microarray fluorescent labelling to similar amounts of starting material. Such a procedure is interesting as the amplification is supposed to be linear and thus to cause less quantitative bias than PCR. However, it is never possible to rule out the possibility of introducing quantitative bias whenever an enzymatic step changing the quantity of material is added to a procedure, especially when a whole population of RNA is concerned. It is indeed commonly admitted that any amplification of the starting material should be avoided whenever possible in gene expression screening and/or gene detection experiments. If one is not to amplify the probes when using fluorescent labelling, the minimum quantity of starting material is much higher than with radioactive labelling (up to 2µg - 10µg of polyA RNA if one is to detect and quantify expression levels of 1/100.000 - 1/300.000 of total mRNA, respectively).

Secondly, fluorescent scanning induces the slide coating to generate relatively high luminous background. This contributes in a large part to the limitation in the overall sensitivity of very low signal detection with such labelling. To this date, this makes this method hardly suitable for the detection of very low expressed messenger RNAs from low amounts of starting tissue without probe amplification. On the opposite, we observed that such a phenomenon is almost absent with radioactive labelling. Therefore, very low hybridisation signals corresponding to very low-expressed mRNAs are directly detectable with this technique, even when the amount of initial sample is very low, as shown by our result for the tyrosine hydroxylase (TH) gene (detection of less than 0.3 pg of mRNA from total brain mRNA).

Third, the present method allows high intrinsic signal detection dynamic, further enhancing the acquisition and analysis of signal, especially on microarrays. In particular, where readout is performed using the MicroImager, the only saturation effect on a particle counter is that of counting rate. In the present case, this rate compared to the detection threshold allows a signal dynamics well into the 10⁴. This makes it possible to analyse all signals of any intensity during one unique acquisition without any signal saturation. As a consequence, all results from a same microarray may be pooled during the gene expression difference analysis, which is critical in terms of controls. On the opposite, the overall dynamics of laser-based fluorescence acquisition is relatively low (10² to 10³) making it necessary to perform several acquisitions of each microarray in order to analyse all hybridisation signals because of signal saturation effect (in a standardised way, one should perform independent laser-readings for low, medium and high signals). The very fact of separating the results of a same single screening experiment makes their analysis more complicated as additional normalisation is needed before pooling them (results of hybridisation and/or modulation of low, medium and highly expressed genes cannot be directly pooled in a normalised way because of the phenomenon of progressive signal fading).

Other aspects and advantages of the present invention will be described in the following examples, which should be regarded as illustrative and not limiting the scope of protection.

### LEGEND TO THE FIGURES

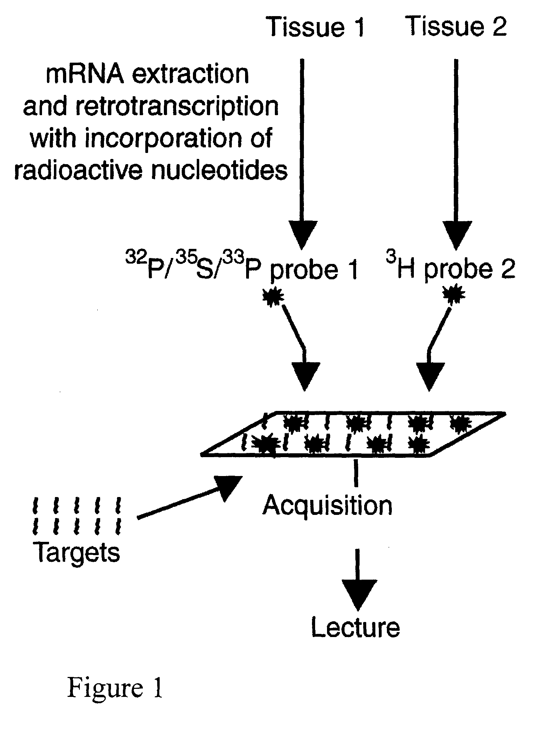

**Figure 1:** Sample preparation method begins with mRNA extraction from cells or tissues. Single-stranded cDNA synthesis with incorporation of radioactive nucleotides allows the labeling of the targets. The labeled targets are denaturated and hybridized to the microarrays overnight. After washing, arrays are submitted to acquisition.

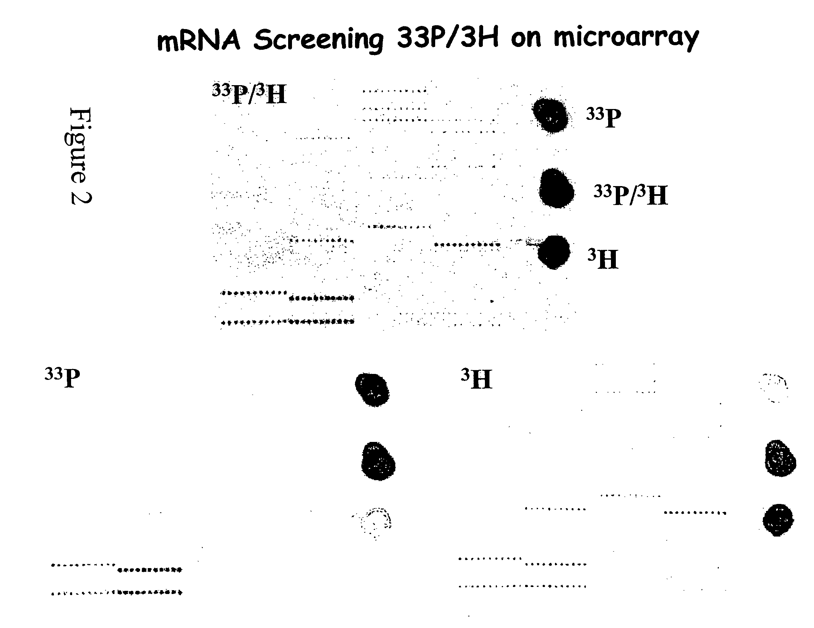

**Figure 2:** Images of hybridisation obtained with 50 ng of ³⁵S-dATP labeled probe and 50 ng of ³H-dCTP labeled probe from two different tissue samples. The targets correspond to PCR products from 300 to 1300 bp spotted on polylysine coated slides. Each target has 10 duplicate.

### EXAMPLES

### Example 1

### Gene array

PCR products from 300 to 1300 bp were purified using the concert nucleic acid purification system and then spotted with an arrayer (Gene machine) on polylysine coated slides (inter-space: 300µm).

### RNA extraction.

Poly (A) RNA were directly isolated from crude extracts of rat brain tissues on magnetic beads (Dynabeads oligo (dT)₂₅, Dynal).

### Sample preparation for hybridization.

cDNA probes corresponding to polyA mRNA were labelled by ³³P dATP (Amersham) or ³H dCTP (Amersham). incorporation during their synthesis. For this, 100 ng to 1 µg of poly (A) were mixed with 0.5 to 5 µg of poly(dT)₂₆, heated to 70°C and progressively cooled to 43°C to ensure annealing of oligo (dT) with the poly (A) tail. Synthesis and probe labelling was then performed in 25 µl in presence of 50 µCi (³³P) dATP , 0.8 mM each dCTP,dTTP and dGTP and 10U AMV reverse transcriptase (Prolabo) for phosphorated probes and 100 µCi (³H) dCTP, 0.8 mM each dCTP,dTTP and dGTP and 10U AMV reverse transcriptase (Prolabo) for tritiated probe. Incubation of the two mixtures was performed at 42°C for 2h. RNA was removed by treatment with 7.5 µl 2M NaOH at 50°C for 30 min followed by neutralization with 7.5 µl of 2.2M acetate. Unincorporated nucleotides were removed on a P10 column (Biorad). For each labelling, probe concentration was adjusted to 10 ng/µl.

The probes were added to the hybridization SSC x3.5, SDS x0.3buffer containing, heated to 95°C for 2 min, cooled to room temperature and then placed on the microarray under a parafilm (Fuji). Each microarray was inserted into a cassette chamber (Telechem). The cassette was submerged into a water bath maintained at 60°C for 16-17h. Following hybridization, the parafilm was removed by deeping the slide in SSC x2, SDS 0.1%, arrays were then rinced in SSC x2 at room temperature for 2 min, and in SSC x0.2 for 2 min.

### Analysis

Real time, digital acquisition of radioactive images was performed with a Micro Imager at a 15 µm spatial resolution and 5 µm pixel size. The arrays are in direct contact imaging through a solid scintillator sheet and image intensified camera. The data were consequently filtered in order to segregate the images corresponding to 3H Beta disintegrations from those corresponding to 33P Beta disintegrations. The results obtained are presented on figure 2. These results are raw data in that no improvement or normalization was performed. The results clearly demonstrate that differently radiolabelled nucleic acid samples can be exposed simultaneously on a nucleic acid array, allowing detection and discrimination of fine gene expression or regulation differences.

### References

1. Mahadevappa M. and Warrington J.A. Nat. Biotechnol. **14**, 1134-1136 (1999)
2. Bertucci F. et al. Human Molecular Genetics, **8,** 1715-1722 (1999)
3. Eberwine et al. Proc. Natl. Acad. Sci., USA **89**, 3010-3014 (1992)

## Claims

1. A method of nucleic acid analysis, comprising contacting at least two differently radiolabelled nucleic acid samples on a nucleic acid array, and analysing nucleic acids in the samples by detecting hybrids formed between the nucleic acids of the samples and the nucleic acid array.

2. A method of nucleic acid analysis, comprising:
e) providing a first nucleic acid sample labelled with a first radiolabel,
f) providing a second nucleic acid sample labelled with a second radiolabel, the second radiolabel being different from the first radiolabel,
g) contacting the first and second nucleic acid samples on a nucleic acid array, and
h) analysing nucleic acids in the samples by detecting hybrids formed between the nucleic acids of the samples and the nucleic acid array.

3. The method of claim 1 or 2, wherein the first nucleic acid sample is a cDNA sample.

4. The method of any one of claims 1 to 3, wherein the second nucleic acid sample is a cDNA sample.

5. The method of claim 3 or 4, wherein the cDNA samples are produced by reverse transcription of RNA populations.

6. The method of claim 5 wherein the cDNA samples are produced by reverse transcription of mRNA populations.

7. The method of claim 5 or 6, wherein the cDNA samples are produced by reverse transcription of total RNAs or total mRNAs of a biological sample.

8. The method of claim 7, wherein the biological sample is a mammalian tissue sample.

9. The method of claim 7, wherein each cDNA sample is produced from RNAs or mRNAs of a different biological sample or from a same type of biological sample in a different physio-pathological condition.

10. The method of claim 1 or 2, wherein at least one of the nucleic acid samples is a gDNA sample.

11. The method of claim 1 or 2, wherein at least one of the nucleic acid samples is a DNA sample.

12. The method of claim 1 or 2, wherein one of the nucleic acid samples comprises one or several control nucleic acids.

13. The method of claim 11, for detecting the presence of a target nucleic acid in the DNA sample.

14. The method of claim 10, for genotyping of a sample.

15. The method of any one of the preceding claims wherein the at least two nucleic acid samples are labelled with radiolabels having a different emission-energy spectra.

16. The method of claim 15, wherein the first nucleic acid sample is labelled with tritium and the second nucleic acid sample is labelled with a radioisotope selected from ³⁵S, ³³P, ³²P and ¹²⁵I.

17. The method of any one of claims 3 to 9 and 15-16, wherein the cDNA samples are radiolabelled during reverse transcription.

18. The method of claim 17, wherein the cDNA samples are radiolabelled by incorporation of radiolabelled nucleotides in their sequence during reverse transcription.

19. The method of any one of the preceding claims, wherein the two samples are contacted simultaneously with the nucleic acid array.

20. The method of any one of the preceding claims, wherein the two samples have essentially the same specific activity.

21. The method of any one of the preceding claims, wherein the nucleic acid array comprises, immobilized on a support, single- or double-stranded nucleic acids selected from oligonucleotides, DNA, RNA, gDNA, gene or genomic fragments, PCR products, PNAs or combinations thereof.

22. The method of any one of the preceding claims, wherein the nucleic acid array comprises nucleic acids immobilized on a support selected from glass, nylon, plastic, silicium, gold and combinations thereof, preferably glass.

23. A method of nucleic acid analysis, comprising:
e) preparing a first cDNA sample labelled with a first radiolabel by reverse transcription of a first RNA population in the presence of a radiolabelled nucleotide labelled with the first radiolabel,
f) preparing a second cDNA sample labelled with a second radiolabel by reverse transcription of a second RNA population in the presence of a radiolabelled nucleotide labelled with the second radiolabel,
g) exposing the first and second cDNA samples to a nucleic acid array, and
h) analysing nucleic acids in the samples by detecting hybrids formed between the nucleic acids of the samples and the nucleic acid array.

24. A method for comparing at least two nucleic acid samples, comprising:
e) labelling a first nucleic acid sample with a first radiolabel,
f) labelling a second nucleic acid sample with a second radiolabel, said first and second radiolabels having a different emission energy spectra,
g) simultaneously exposing at least a portion of said differently radiolabelled nucleic acid samples to a nucleic acid array under conditions allowing hybridisation to occur, and
h) comparing the nucleic acid samples by analysing hybridisation pattern thereof.

25. The method of claim 24, wherein the nucleic acid samples exhibit essentially similar specific activities.

26. The method of claim 24 or 25, wherein the nucleic acid samples are cDNA samples prepared from RNA samples without amplification, and labelled during reverse transcription.

27. The method of any one of the preceding claims, wherein assessing hybrid formation comprises (i) washing the unbound nucleic acids and (ii) detecting radioactivity on the sample.

28. The use of at least two differently radiolabelled nucleic acid samples for in vitro gene expression analysis or gene detection on a nucleic acid array.

29. A method of any one of claims 1 to 27, further comprising contacting the nucleic acid array(s) with a non-radioactive nucleic acid sample to detect additional target nucleic acid(s).

30. A kit for implementing a method according to any one of claims 1 to 27 and 29, comprising the reagents, supports and/or protocols for labelling, hybridisation and/or readout.

31. A method of preparing a radiolabelled nucleic acid sample, comprising:
(a) obtaining RNAs from a biological sample, preferably mRNAs, more preferably using polyT-coated support, and
(b) reverse transcribing the RNAs in the presence of a tritiated nucleotide, in order to produce tritiated cDNAs having incorporated in their sequence tritiated nucleotides.

32. The use of tritium for detecting nucleic acid hybridization on a nucleic acid array.

33. A method of nucleic acid analysis comprising a hybridisation of a nucleic acid sample on a nucleic acid array, wherein the nucleic acid sample is radiolabelled with tritium.
